# EUROPÄISCHE PATENTSCHRIFT

(11) **EP 2 318 342 B1**
(45) Veröffentlichungstag und Bekanntmachung des Hinweises auf die Patenterteilung: **29.08.2012**
(21) Anmeldenummer: 09772082.5
(22) Anmeldetag: 12.06.2009
(51) Int. Cl.: C07C 29/141, C07C 31/20

(54) **VERFAHREN ZUR HERSTELLUNG VON NEOPENTYLGLYKOL**
METHOD OF PRODUCING NEOPENTYL GLYCOL
PROCÉDÉ DE PRÉPARATION DE NÉOPENTYLGLYCOL

(30) Priorität: 02.07.2008 DE 102008031338
(43) Veröffentlichungstag der Anmeldung: 11.05.2011
(73) Patentinhaber: OXEA GmbH, 46147 Oberhausen (DE)
(72) Erfinder: SCHALAPSKI, Kurt, 46147 Oberhausen (DE); WEBER, Tonia, 64293 Darmstadt (DE); KREICKMANN, Thorsten, 46149 Oberhausen (DE); HEYMANNS, Peter, 45147 Essen (DE); LUKAS, Rainer, 45133 Essen (DE); SCHULZ, Rolf-Peter, 65830 Kriftel (DE)
(86) Internationale Anmeldenummer: PCT/EP2009/004268
(87) Internationale Veröffentlichungsnummer: WO 2010/000382

(56) Entgegenhaltungen:
- WO-A1-99/35112
- WO-A1-2004/092097
- US-B1- 6 268 539

## Beschreibung

Die vorliegende Erfindung betrifft ein Verfahren zur Herstellung von Neopentylglykol durch Hydrierung von Hydroxypivalinaldehyd in der Flüssigphase an einem Nickel haltigen Katalysator in Gegenwart von mehr als 15 Gew.-% Wasser, bezogen auf das Einsatzgemisch.

Mehrwertige Alkohole oder Polyole besitzen als Kondensationskomponente zum Aufbau von Polyestern oder Polyurethanen, Kunstharzlacken, Schmiermitteln und Weichmachern eine erhebliche wirtschaftliche Bedeutung. Hierbei sind besonders solche mehrwertigen Alkohole von Interesse, die durch eine gemischte Aldoladdition von Formaldehyd mit iso- oder n-Butyraldehyd erhalten werden. Bei der Aldoladdition zwischen Formaldehyd und dem entsprechenden Butyraldehyd bildet sich zunächst eine aldehydische Zwischenstufe, die anschließend zum mehrwertigen Alkohol reduziert werden muss. Ein technisch wichtiges Beispiel für einen solchen mehrwertigen Alkohol, der nach diesem Verfahren zugänglich ist, ist Neopentylglykol [NPG, 2,2-Dimethylpropandiol-(1,3)], der durch Mischaldolisierung von Formaldehyd und Isobutyraldehyd erhalten wird. Die Aldoladditionsreaktion wird mit äquimolaren Mengen in Gegenwart basischer Katalysatoren, beispielsweise Alkalihydroxiden oder aliphatischen Aminen, durchgeführt und liefert zunächst das isolierbare Zwischenprodukt Hydroxypivalinaldehyd (HPA). Dieses Zwischenprodukt kann anschließend mit überschüssigem Formaldehyd entsprechend der Cannizzaro-Reaktion in Neopentylglykol unter Bildung eines Äquivalents eines Formiatsalzes überführt werden. Bei dieser Ausgestaltung des Reduktionsschrittes fällt daher das Formiatsalz als Koppelprodukt an. Technisch umgesetzt wird aber auch die katalytische Hydrierung von Hydroxypivalinaldehyd in der Gas- und Flüssigphase am Metallkontakt. Als geeignete Hydrierkatalysatoren haben sich gemäß EP 0 278 106 A1 Nickelkatalysatoren erwiesen, die gegebenenfalls noch weitere aktive Metalle wie Chrom oder Kupfer und darüber hinaus Aktivatoren enthalten können. Das rohe Aldolisierungsgemisch wird anschließend ohne vorherige Trennung in seine Bestandteile oder Abtrennung einzelner Komponenten katalytisch hydriert. Da üblicherweise Formaldehyd als wässrige Lösung eingesetzt wird, beispielsweise als 37 Gew.-%ige Lösung, ist in dem zu hydrierenden Aldolisierungsgemisch Wasser zugegen. Das erhaltene rohe Hydrierprodukt kann dann gemäß der Lehre nach EP 0 278 106 A1 destillativ aufgearbeitet werden.

Ein weiteres Verfahren zur Hydrierung von Hydroxypivalinaldehyd zu Neopentylglykol in der Flüssigphase in Gegenwart von Nickelkatalysatoren ist aus WO 99/035112 A1 bekannt. Besonders wird auf den schädlichen Einfluss einer zu hohen Wassermenge auf die Stabilität des Nickelkatalysators während des Hydrierprozesses hingewiesen. Es wird über eine Katalysatorschädigung und auch über einen Selektivitätsrückgang zu Lasten von Neopentylglykol durch die Gegenwart von Wasser berichtet. WO 99/035112 A1 schlägt daher vor, in der Hydrierung von Hydroxypivalinaldehyd zu Neopentylglykol die Wassermenge auf weniger als 15 Gew.-% zu begrenzen. Auch soll nach dem bekannten Verfahren die Hydriertemperatur von 100°C nicht überschritten werden, da es bei der Anwendung höherer Hydriertemperaturen in Gegenwart von Nickelkatalysatoren zu einer verstärkten Nebenproduktbildung, wie der Bildung von Neopentylglykol-monoisobutyrat oder Neopentylglykolmonohydroxypivalinsäureester kommt.

Auch WO 98/17614 A1 behandelt die Hydrierung von Hydroxypivalinaldehyd zu Neopentylglykol nach dem Flüssigphasenverfahren in Gegenwart von Nickelkatalysatoren. Nach dem bekannten Prozess wird zunächst Isobutyraldehyd mit einer wässrigen Formaldehydlösung in Gegenwart eines tertiären Alkylamins zu einem Hydroxypivalinaldehyd enthaltenden Rohgemisch umgesetzt, das anschließend einer Extraktion mit einem aliphatischen Alkohol unterzogen wird. Aus der organischen Phase werden die niedrig siedenden Anteile abdestilliert und die höher siedenden Anteile, die Hydroxypivalinaldehyd enthalten, hydriert. Zur Aufarbeitung wird das Hydrierprodukt mit Wasser extrahiert, wobei Neopentylglykol in die wässrige Phase übergeht. Aus der wässrigen Phase wird dann Neopentylglykol destillativ isoliert. Durch den der Hydrierstufe vorgeschalteten Extraktions- und Destillationsschritt wird die Menge des in der Hydrierstufe anwesenden Wassers vermindert. Nach dem bekannten Verfahren soll die Hydrierstufe in einem Temperaturbereich von 120°C bis 180°C durchgeführt werden.

Gemäß US 6,268,539 B1 wird das unter Triethylamin-Katalyse erhaltene Aldolisierungsprodukt aus der Umsetzung von Isobutyraldehyd und einer wässrigen Formaldehydlösung zunächst destilliert. Der angefallene Wasser haltige Destillationsrückstand wird anschließend bei 70 bis 120°C in Gegenwart von Raney-Nickel hydriert, das Molybdän als Promotor enthält. Das bekannte Flüssigphasenverfahren ist durch den Einsatz eines speziellen Saugrührers charakterisiert, der eine intensive Vermischung zwischen der flüssigen und gasförmigen Phase sicherstellt. Durch diese spezielle Reaktorauslegung sind nur geringe Hydrierdrücke im Bereich von 0,55 bis 12,4 MPa erforderlich.

Auch nach der aus der EP 0 395 681 B1 bekannten Reaktionsführung wird die Flüssigphasenhydrierung von Hydroxypivalinaldehyd in Gegenwart von Raney-Nickel unter Verwendung eines speziellen Reaktordesigns durchgeführt, bei dem Wasserstoffgas durch das flüssige Reaktionsgut intensiv geleitet wird. Durch diesen Stripp-Effekt werden Spuren des als Aldolisierungskatalysator verwendeten tertiären Amins und seine Verbindungen entfernt, die die Zersetzung des Hydroxypivalinaldehyds in der Hydrierstufe-fördern. Auf die Anwendung hoher Drücke kann nach der Lehre von EP 0 395 681 B1 verzichtet werden. Das in die Hydrierstufe eingesetzte Rohgemisch enthält 10 bis 35 Gew.-% Wasser.

Für die Flüssigphasenhydrierung von Hydroxypivalinaldehyd zu Neopentylglykol in Gegenwart von Nickelkatalysatoren ist entweder eine besondere Reaktorauslegung erforderlich, oder es werden nur geringe Wassergehalte in dem rohen Hydroxypivalinaldehyd für den Einsatz zur Hydrierung zugelassen, um Hydroxypivalinaldehyd bei hohem Umsatz mit hoher Selektivität zu Neopentylglykol umzusetzen. Gegebenenfalls muss der rohe Hydroxypivalinaldehyd zunächst einer zusätzlichen Extraktion und Destillation unterzogen werden, um den Wassergehalt in dem zu hydrierenden Produkt zu vermindern.

Es ist jedoch wünschenswert, das Umsetzungsprodukt aus der Alkylamin katalysierten Aldoladdition von Isobutyraldehyd mit einer wässrigen Formaldehydlösung direkt und ohne Reinigungsschritte in Gegenwart eines gängigen, technisch verfügbaren Nickelkatalysators in der Flüssigphase zu hydrieren.

Es besteht daher die Aufgabe, einen Prozess zu entwickeln, der technisch einfach durchzuführen ist und mit wirtschaftlich vertretbaren Mitteln die Gewinnung von Neopentylglykol durch Alkylamin katalysierte Aldoladdition ermöglicht.

Die Erfindung besteht daher in einem kontinuierlichen Verfahren zur Herstellung von Neopentylglykol durch Addition von Isobutyraldehyd und Formaldehyd in Gegenwart eines tertiären Alkylamins als Katalysator zum Hydroxypivalinaldehyd mit nachfolgender Hydrierung, dadurch gekennzeichnet, dass die Hydrierung in einem Rohrreaktor ohne Einbauten und ohne Rührvorrichtungen durchgeführt wird und bei einer Temperatur von 110 bis 180°C und bei einem Druck von 6 bis 18 MPa in Gegenwart von Nickelkatalysatoren in der homogenen flüssigen Phase erfolgt, die einen aliphatischen Alkohol als organisches Lösungsmittel- oder Verdünnungsmittel in einer Menge von 15 bis 27 Gew.-%, bezogen auf den organischen Anteil in dem Einsatzgemisch, und Wasser in einer Menge von mehr als 15 bis 25 Gew.-%, bezogen auf die gesamte Einsatzmenge, enthält.

Überraschender Weise wurde gefunden, dass bei Wassergehalten innerhalb eines Bereiches von mehr als 15 bis 25 Gew.-%, vorzugsweise 18 bis 22 Gew.-% bezogen auf die gesamte Einsatzmenge, und beim Einstellen einer Hydriertemperatur von 110 bis 180°C, vorzugsweise von 110 bis 140°C die selektive Hydrierung von Hydroxypivalinaldehyd zu Neopentylglykol gelingt und die sehr selektive Spaltung von Hochsiedern, die sich während der Umsetzung von Isobutyraldehyd mit Formaldehyd bilden, zu Neopentylglykol möglich ist. Bei den Hochsiedern handelt es sich um Sauerstoff haltige Verbindungen, wie Ester oder cyclische Acetale, in denen Äquivalente des Neopentylglykols chemisch gebunden sind. In den Hochsiedern ist der Anteil an Mono- und Di-Isobuttersäureestern des Neopentylglykols sowie an dem aus Hydroxypivalinaldehyd nach der Tischtschenko-Reaktion gebildeten Disproportionierungsprodukt Neopentylglykolmonohydroxypivalinsäureester besonders hoch. Durch die erfindungsgemäße Einstellung des Hydrierschritts in Bezug auf den Wassergehalt im Einsatzprodukt und durch die genaue Wahl der Hydriertemperatur können bereits im Einsatzprodukt vorhandene Hochsieder wirksam zu Neopentylglykol gespalten und ihre Bildung während der Hydrierreaktion unterdrückt werden, im Vergleich zu einer Arbeitsweise, bei der man ein Einsatzprodukt mit einem Wassergehalt von weniger als 15 Gew.-% verwendet oder bei einer Hydriertemperatur von weniger als 110°C arbeitet.

Bei zu geringen Wassergehalten im Einsatzprodukt beobachtet man keinen vorteilhaften Effekt mehr auf die Verminderung des Hochsiederanteils und bei zu niedrigen Hydriertemperaturen wird Hydroxypivalinaldehyd nur unvollständig hydriert. Bei zu hohen Wassergehalten wird wertvolles Reaktorvolumen unnötig belegt und nicht ausgenutzt. Bei zu hohen Hydriertemperaturen tritt ebenfalls verstärkt eine Zersetzung des als Aldolisierungskatalysator eingesetzten tertiären Alkylamins ein, die zu schwer abtrennbaren Folgeprodukten führt und daher unerwünscht ist.

Die Aldoladdition von Isobutyraldehyd und einer wässrigen Formaldehydlösung erfolgt in Gegenwart von tertiären Alkylaminen als Aldoladditionskatalysator, beispielsweise in Gegenwart von Trimethyl-, Triethyl-, Tri-n-propyl-, Tri-iso-propyl-, Methyl-diethyl-, Methyl-diisopropylamin oder Tributylamin. Als besonders geeignete Katalysatoren haben sich Triethylamin und Tri-n-propylamin bewährt.

Die Aldehyde können im molaren Verhältnis umgesetzt werden, jedoch ist es auch möglich, einen der beiden Reaktionspartner im Überschuss anzuwenden. Man setzt Formaldehyd als wässrige Lösung ein, der Aldehydgehalt beträgt üblicherweise von 20 bis 50 Gew.-%. Die Reaktion erfolgt bei Temperaturen zwischen 20 und 100°C, zweckmäßig arbeitet man bei 80 bis 95°C. Im Allgemeinen wird die Reaktion bei Normaldruck durchgeführt, jedoch kann man auch erhöhten Druck anwenden. Das als Aldoladditionskatalysator verwendete tertiäre Alkylamin ist in dem Reaktionsgemisch in einer Menge von 1 bis 20, vorzugsweise 2 bis 12 Mol.-%, bezogen auf Isobutyraldehyd, enthalten.

Neben dem Wasser aus der wässrigen Formaldehydlösung und geringen Anteilen an Methanol, das ebenfalls in der wässrigen Formaldehydlösung enthalten ist, wird gegebenenfalls der Reaktionsmischung noch Isobutanol als Verdünnungsmittel zugesetzt. Der Isobutanolzusatz ist nicht zwingend erforderlich, falls jedoch Isobutanol zugesetzt wird, liegt sein Gehalt in der Reaktionsmischung im Bereich von 10 bis 20 Gew.-%, bezogen auf das gesamte Reaktionsgemisch. Weitere Lösungs- und Verdünnungsmittel sind nicht erforderlich.

Die praktische Durchführung der Additionsreaktion erfolgt in einem Rührkessel oder in einem Reaktionsrohr, das zur besseren Durchmischung der Reaktanten mit Füllkörpern beschickt ist. Die Umsetzung verläuft exotherm, sie kann durch Erhitzen beschleunigt werden.

Das nach der Aldoladdition anfallende Rohgemisch wird ohne vorherige Trennung in seine Bestandteile oder Abtrennung einzelner Komponenten katalytisch hydriert. Wesentlich für die erfindungsgemäße Hydrierung des Hydroxypivalinaldehyd enthaltenden Reaktionsgemischs ist das Einhalten eines definierten Wassergehalts, einer definierten Hydriertemperatur und eines bestimmten Reaktionsdrucks. Reicht die durch die Verwendung der wässrigen Formaldehydlösung eingetragene Wassermenge nicht aus, um den geforderten Wasseranteil zu gewährleisten, ist vor dem Einsatz in dem Hydrierreaktor Wasser dem Rohprodukt zuzusetzen.

Die Hydrierung erfolgt ebenfalls in Gegenwart eines aliphatischen Alkohols, der mit dem Aldolisierungsrohprodukt mischbar ist. Als geeignete aliphatische Alkohole haben sich lineare oder verzweigte Alkohole mit 1 bis 5 Kohlenstoffatomen, beispielsweise Methanol, Ethanol, n-Propanol, iso-Propanol, n-Butanol, iso-Butanol oder Mischungen davon, erwiesen. Besonders zweckmäßig verwendet man Isobutanol, da Restmengen von Isobutyraldehyd zu Isobutanol hydriert werden. Falls man Isobutanol bereits in der Aldoladditionsstufe als Verdünnungsmittel einsetzt, liegt in der Hydrierstufe schon ein Lösungsmittel vor. Geringe Mengen an Methanol, die über die wässrige Formaldehydlösung eingetragen werden, sind ebenfalls zugegen. Der Anteil des aliphatischen Alkohols als organisches Lösungs- oder Verdünnungsmittel beträgt 15 bis 27 Gew.-%, vorzugsweise 15 bis 18 Gew.-%, bezogen auf den organischen Anteil in dem Einsatzgemisch. Durch den Zusatz des Verdünnungs- oder Lösungsmittels wird eine ausreichende Löslichkeit des Hydroxypivalinaldehyds in der flüssigen Phase während der Hydrierstufe sichergestellt sowie das Ausfallen von Hydroxypivalinaldehyd verhindert und die Homogenität der flüssigen Phase gewährleistet.
Das gesamte, für die Hydrierung verwendete Einsatzgemisch ist homogen und enthält somit mehr als 15 und bis 25 Gew.-% Wasser und als Rest auf 100 Gew.-% einen organischen Anteil, der wiederum 15 bis 27 Gew.-% eines aliphatischen Alkohols enthält.

Das erhaltene, Hydroxypivalinaldehyd enthaltende Rohgemisch wird ohne weitere Reinigungs- und Aufarbeitungsschritte hydriert.

Die Hydrierung des rohen Hydroxypivalinaldehyds wird bei einer Temperatur von 110 bis 180°C, vorzugsweise von 110 bis 140°C, in der flüssigen Phase in Gegenwart von Nickelkatalysatoren durchgeführt. Der Reaktionsdruck beträgt 6 bis 18 MPa, vorzugsweise 8 bis 15 MPa. Bei niedrigeren Reaktionsdrücken wird keine zufriedenstellende Hydrierung des Hydroxypivalinaldehyds mehr beobachtet.

Nickel als katalytisch aktives Metall kann auf einem Träger aufgebracht werden, im Allgemeinen in einer Menge von etwa 5 bis 70 Gew.-%, vorzugsweise etwa 10 bis etwa 65 Gew.-% und insbesondere etwa 20 bis 60 Gew.%, jeweils bezogen auf das Gesamtgewicht des Katalysators. Als Katalysatorträger eignen sich alle herkömmlichen Trägermaterialien, zum Beispiel Aluminiumoxid, Aluminiumoxidhydrate in ihren verschiedenen Erscheinungsformen, Siliciumdioxid, Polykieselsäuren (Kieselgele) einschließlich Kieselgur, Kieselxerogele, Magnesiumoxid, Zinkoxid, Zirconiumoxid und Aktivkohle. Neben den Hauptkomponenten Nickel und Trägermaterial können die Katalysatoren noch Zusatzstoffe in untergeordneten Mengen enthalten, die zum Beispiel der Verbesserung ihrer Hydrieraktivität und/oder ihrer Standzeit und/oder ihrer Selektivität dienen. Derartige Zusatzstoffe sind bekannt, zu ihnen gehören zum Beispiel die Oxide des Natrium, Kaliums, Magnesiums, Calciums, Bariums, Zinks, Aluminiums, Zirconiums und Chroms. Sie werden dem Katalysator im Allgemeinen in einem Anteil von insgesamt 0,1 bis 50 Gew.-Teile, bezogen auf 100 Gew.-Teile Nickel, zugesetzt.

Aber auch Raney-Nickel als Träger freier Katalysator kann verwendet werden.

Die Hydrierung wird in der Flüssigphase kontinuierlich durchgeführt, beispielsweise an fest angeordneten Katalysatoren nach der Rieselfahrweise oder Sumpffahrweise.

Bei der kontinuierlichen Fahrweise hat sich eine Katalysatorbelastung V/Vh, ausgedrückt in Durchsatzvolumen pro Katalysatorvolumen und Zeit, von 0,3 bis 2,0 h⁻¹, vorzugsweise 0,8 bis 1,2 h⁻¹, als zweckmäßig erwiesen.

Eine höhere Belastung des Nickelkatalysators ist zu vermeiden, weil die Ausgangsverbindung Hydroxypivalinaldehyd dann nicht mehr vollständig hydriert wird und eine verstärkte Nebenproduktbildung beobachtet wird.

Die Hydrierung wird kontinuierlich in flüssiger Phase in einem Rohrreaktor an fest angeordneten Katalysatoren durchgeführt. Unter einem Rohrreaktor ist auch ein Bündel von mehreren eng parallel geschalteten Rohren zu verstehen. Die Hydrierung von Hydroxypivalinaldehyd erfolgt in einem Rohrreaktor ohne Einbauten und ohne Rührvorrichtungen.

Die Hydrierung erfolgt vorzugsweise mit reinem Wasserstoff. Es können jedoch auch Gemische verwendet werden, die freien Wasserstoff und daneben unter den Hydrierbedingungen inerte Bestandteile enthalten.

Die Gewinnung des reinen Neopentylglykols aus dem hydrierten Reaktionsgemisch erfolgt nach konventionellen Destillationsverfahren. Dabei abgetrenntes Lösungs- oder Verdünnungsmittel kann wieder in die Aldoladditionsstufe und/oder Hydrierstufe zurückgeführt werden.

Nach dem erfindungsgemäßen Hydrierverfahren wird Hydroxypivalinaldehyd bei hohem Umsatz mit hoher Selektivität in Neopentylglykol umgewandelt. Bemerkenswert ist der geringe Anteil an Hochsiedern nach Hydrierung.

Durch das erfindungsgemäße Hydrierverfahren wird die Ausgangsverbindung Hydroxypivalinaldehyd bei hohem Umsatz sehr selektiv zu Neopentylglykol hydriert und es werden die in der Aldoladditionsvorstufe gebildeten Hochsieder wirksam gespalten und ihre Bildung in der Hydrierstufe nachhaltig unterbunden. Auch wird die Spaltung des tertiären Alkylamins in flüchtige, Stickstoff haltige Verbindungen, die zu unerwünschten Verunreinigungen führen und die bei der anschließenden destillativen Aufarbeitung nur schwierig abzutrennen sind und die bei der Weiterverarbeitung von Neopentylglykol stören, unterdrückt.

Im Folgenden wird das erfindungsgemäße Verfahren anhand einiger Beispiele näher erläutert.

### Versuchsaufbau

Die Flüssigphasenhydrierung erfolgte an einem kommerziellen, geträgerten Nickelkatalysator im Rohrreaktor in Sumpffahrweise. Das Katalysatorvolumen betrug 1,8 Liter. Das Hydroxypivalinaldehyd enthaltende rohe Aldoladditionsprodukt und Wasserstoff wurden am Sumpf des Rohrreaktors kontinuierlich zugeführt. Über den Kopf des Rohrreaktors entnahm man das Hydriergut, leitete es in einen Hochdruckabscheider und führte es daraus über eine Standregelung in eine drucklose Vorlage. Die Hydriertemperatur, der Wasserstoffdruck sowie die Katalysatorbelastung wurde gemäß den Bedingungen der nachfolgenden Tabellen eingestellt. Das für die Hydrierversuche verwendete rohe, Hydroxypivalinaldehyd enthaltende Aldoladditionsprodukt wies folgende typische Zusammensetzung auf.

Organischer Anteil (gaschromatographisch ermittelt, Angaben in Prozent):

| | |
|---|---|
| Leichtsieder | 0,1 |
| Isobutanal | 2,0 |
| Methanol | 0,9 |
| Zwischenlauf | 7,5 |
| Isobutanol | 21,1 |
| HPA | 62,3 |
| NPG | 2,2 |
| TE | 2,9 |
| Nachlauf | 1,0 |
| | |
| Wasser | 18,5 Gew.% bezogen auf die gesamte Einsatzmischung |

| | |
|---|---|
| HPA = Hydroxypivalinaldehyd NPG = Neopentylglykol TE = Tischtschenkoester / Di-isobuttersäure-NPG-Ester | |

Bei den nachfolgend angegebenen Analysendaten für die Einsatzströme wurden die kritischen Gehalte für die als Verdünnungsmittel dienenden aliphatischen Alkohole sowie der Wassergehalt ausgewiesen. Bei der Analyse der Hydrierausträge wurden die Restgehalte an HPA sowie an Esterverbindungen und der NPG-Gehalt angegeben.

Flüssigphasenhydrierung von HPA bei einer Hydriertemperatur von 130°C

**Tabelle 1: Druck 8 MPa**

| **Versuch** | **VNh [h⁻¹]** | **Analyse der Einsatzströme, gaschromatographisch ermittelt, in Prozent *)** | | | **Analyse der Hydrierausträge, gaschromatographisch ermittelt, in Prozent** | | | | |
|---|---|---|---|---|---|---|---|---|---|
| | | **i-Butanol** | **Methanol** | **Wasser **) Gew.%** | **HPA** | **TE** | **NPG** | **Leichtsieder** | **Hochsieder** |
| 1 | 0,27 | 19,6 | 1,0 | 18,9 | 0,1 | 6,2 | 67,1 | 25,0 | 1,6 |
| 2 | 0,53 | 21,0 | 0,9 | 15,7 | 0,1 | 5,2 | 65,0 | 28,2 | 1,5 |
| 3 | 0,90 | 23,9 | 1,2 | 15,2 | 1,0 | 3,9 | 62,8 | 31,1 | 1,2 |

**Tabelle 2: Druck 6 MPa**

| **Versuch** | **VNh [h⁻¹]** | **Analyse der Einsatzströme, gaschromatographisch ermittelt, in Prozent *)** | | | **Analyse der Hydrierausträge, gaschromatographisch ermittelt, in Prozent** | | | | |
|---|---|---|---|---|---|---|---|---|---|
| | | **i-Butanol** | **Methanol** | **Wasser **) Gew.%** | **HPA** | **TE** | **NPG** | **Leichtsieder** | **Hochsieder** |
| 4 | 1,00 | 22,9 | 1,0 | 18,4 | 0,4 | 4,0 | 62,9 | 31,3 | 1,4 |
| 5 (Vergleich) | 0,90 | 23,3 | 0,9 | 14,6 | 0,1 | 4,4 | 61,8 | 32,2 | 1,5 |

**Tabelle 3: Druck 14 MPa**

| **Versuch** | **VNh [h⁻¹]** | **Analyse der Einsatzströme, gaschromatographisch ermittelt, in Prozent *)** | | **Analyse** | **Analyse der Hydrierausträge, gaschromatographisch ermittelt, in Prozent** | | | | |
|---|---|---|---|---|---|---|---|---|---|
| | | **i-Butanol** | **Methanol** | **Wasser **) Gew.%** | **HPA** | **TE** | **NPG** | **Leichtsieder** | **Hochsieder** |
| 6 | 1,00 | 22,9 | 1,0 | 16,1 | 0,2 | 3,7 | 63,3 | 31,2 | 1,6 |
| 7 | 1,33 | 22,1 | 1,1 | 18,5 | 0,4 | 2,5 | 65,0 | 30,7 | 1,4 |
| 8 (Vergleich) | 1,50 | 22,7 | 1,1 | 14,4 | 1,6 | 3,1 | 62,0 | 31,5 | 1,8 |

**Tabelle 4: Druck 4 MPa**

| **Versuch** | **V/Vh [h⁻¹]** | **Analyse der Einsatzströme, gaschromatographisch ermittelt, in Prozent*)** | | | **Analyse der Hydrierausträge, gaschromatographisch ermittelt, in Prozent** | | | | |
|---|---|---|---|---|---|---|---|---|---|
| | | **i-Butanol** | **Methanol** | **Wasser **) Gew.%** | **HPA** | **TE** | **NPG** | **Leichtsieder** | **Hochsieder** |
| 9 (Vergleich) | 0,90 | 23,6 | 1,6 | 16,9 | 4,7 | 4,5 | 55,7 | 33,3 | 1,8 |

| | | | | | | | | | |
|---|---|---|---|---|---|---|---|---|---|
| *) bezogen auf den organischen Anteil in dem gesamten Einsatzgemisch **) Wasser in Gew.-%, bezogen auf die gesamte Einsatzmischung | | | | | | | | | |

Wie ein Vergleich der Versuchdaten zeigt, erhöht sich mit steigendem Wassergehalt in der Einsatzmischung auch der Anteil an dem gewünschten NPG in dem Hydrieraustrag. Wird beispielsweise ausgehend von dem Versuch 3 der Wassergehalt unter die kritische Grenze von 15 Gew.-% eingestellt (Vergleichsversuch 5), erniedrigt sich der NPG-Gehalt in dem Hydrieraustrag. Dieser Gang zeigt auch bei den Versuchen 6, 7 und 8 (Vergleich), bei denen mit fallendem Wassergehalt im Einsatzprodukt ebenfalls der Gehalt an NPG im Hydrieraustrag abnimmt. Der im Vergleichsbeispiel 9 gewählte Druck reicht für einen zufriedenstellenden HPA-Umsatz nicht mehr aus.

## Patentansprüche

1. Kontinuierliches Verfahren zur Herstellung von Neopentylglykol durch Addition von Isobutyraldehyd und Formaldehyd in Gegenwart eines tertiären Alkylamins als Katalysator zum Hydroxypivalinaldehyd mit nachfolgender Hydrierung, **dadurch gekennzeichnet, dass** die Hydrierung in einem Rohrreaktor ohne Einbauten und ohne Rührvorrichtungen durchgeführt wird und bei einer Temperatur von 110 bis 180°C und bei einem Druck von 6 bis 18 MPa in Gegenwart von Nickelkatalysatoren in der homogenen flüssigen Phase erfolgt, die einen aliphatischen Alkohol als organisches Lösungsmittel- oder Verdünnungsmittel in einer Menge von 15 bis 27 Gew.-%, bezogen auf den organischen Anteil in dem Einsatzgemisch, und Wasser in einer Menge von mehr als 15 bis 25 Gew.-%, bezogen auf die gesamte Einsatzmenge, enthält.

2. Verfahren gemäß Anspruch 1, **dadurch gekennzeichnet, dass** die Hydrierung bei einer Temperatur von 110 bis 140°C und bei einem Druck von 8 bis 15 MPa durchgeführt wird.

3. Verfahren gemäß Anspruch 1 oder 2, **dadurch gekennzeichnet, dass** die homogene flüssige Phase Wasser in einer Menge von 18 bis 22 Gew.-%, bezogen auf die gesamte Einsatzmenge, enthält.

4. Verfahren gemäß einem oder mehreren der Ansprüche 1 bis 3, **dadurch gekennzeichnet, dass** man als tertiäres Alkylamin Triethylamin oder Tri-n-propylamin verwendet.

5. Verfahren gemäß einem oder mehreren der Ansprüche 1 bis 4, **dadurch gekennzeichnet, dass** man als aliphatischen Alkohol lineare oder verzweigte Alkohole mit 1 bis 5 Kohlenstoffatomen verwendet.

6. Verfahren gemäß Anspruch 5, **dadurch gekennzeichnet, dass** man Methanol, Ethanol, n-Propanol, iso-Propanol, n-Butanol, iso-Butanol oder Mischungen davon verwendet.

7. Verfahren gemäß einem oder mehreren der Ansprüche 1 bis 6, **dadurch gekennzeichnet, dass** der Nickelkatalysator ein Trägermaterial enthält.

8. Verfahren gemäß einem oder mehreren der Ansprüche 1 bis 7, **dadurch gekennzeichnet, dass** der Nickelkatalysator als Zusatzstoffe Oxide des Natriums, Kaliums, Magnesiums, Calciums, Bariums, Zinks, Aluminiums, Zirconiums, Chroms oder Mischungen davon enthält.

## Claims

1. A continuous process for preparing neopentyl glycol by addition of isobutyraldehyde and formaldehyde in the presence of a tertiary alkylamine as a catalyst to give hydroxypivalaldehyde with subsequent hydrogenation, **characterized in that** the hydrogenation is performed in a tubular reactor without internals and without stirrer apparatus and is effected at a temperature of 110 to 180°C and at a pressure of 6 to 18 MPa in the presence of nickel catalysts in the homogeneous liquid phase which contains, as an organic solvent or diluent, an aliphatic alcohol in an amount of 15 to 27% by weight, based on the organic component in the starting mixture, and water in an amount of more than 15 to 25% by weight, based on the total amount used.

2. The process as claimed in claim 1, **characterized in that** the hydrogenation is performed at a temperature of 110 to 140°C and at a pressure of 8 to 15 MPa.

3. The process as claimed in claim 1 or 2, **characterized in that** the homogeneous liquid phase contains water in an amount of 18 to 22% by weight based on the total amount used.

4. The process as claimed in one or more of claims 1 to 3, **characterized in that** the tertiary alkylamine used is triethylamine or tri-n-propylamine.

5. The process as claimed in one or more of claims 1 to 4, **characterized in that** the aliphatic alcohol used comprises linear or branched alcohols having 1 to 5 carbon atoms.

6. The process as claimed in claim 5, **characterized in that** methanol, ethanol, n-propanol, isopropanol, n-butanol, isobutanol or mixtures thereof are used.

7. The process as claimed in one or more of claims 1 to 6, **characterized in that** the nickel catalyst comprises a support material.

8. The process as claimed in one or more of claims 1 to 7, **characterized in that** the nickel catalyst comprises, as additives, oxides of sodium, of potassium, of magnesium, of calcium, of barium, of zinc, of aluminum, of zirconium, of chromium or mixtures thereof.

## Revendications

1. Procédé continu pour la production de néopentylglycol par addition d'isobutyraldéhyde et formaldéhyde en présence d'une alkylamine tertiaire en tant que catalyseur, conduisant à l'hydroxypivalaldéhyde avec hydrogénation subséquente, **caractérisé en ce que** l'hydrogénation est effectuée dans un réacteur tubulaire sans inserts et sans dispositifs d'agitation et à une température de 110 à 180 °C et sous une pression de 6 à 18 MPa en présence de catalyseurs au nickel, dans la phase liquide homogène qui contient un alcool aliphatique en tant que solvant ou diluant organique, en une quantité de 15 à 27 % en poids, par rapport à la fraction organique dans le mélange d'alimentation, et de l'eau en une quantité de plus de 15 à 25 % en poids, par rapport à la quantité totale d'alimentation.

2. Procédé selon la revendication 1, **caractérisé en ce que** l'hydrogénation est effectuée à une température de 110 à 140 °C et sous une pression de 8 à 15 MPa.

3. Procédé selon la revendication 1 ou 2, **caractérisé en ce que** la phase liquide homogène contient de l'eau en une quantité de 18 à 22 % en poids, par rapport à la quantité totale d'alimentation.

4. Procédé selon une ou plusieurs des revendications 1 à 3, **caractérisé en ce qu'**on utilise comme alkylamine tertiaire la triéthylamine ou la tri-n-propylamine.

5. Procédé selon une ou plusieurs des revendications 1 à 4, **caractérisé en ce qu'**on utilise comme alcool aliphatique des alcools linéaires ou des alcools ramifiés ayant de 1 à 5 atomes de carbone.

6. Procédé selon la revendication 5, **caractérisé en ce qu'**on utilise le méthanol, l'éthanol, le n-propanol, l'isopropanol, le n-butanol, l'isobutanol ou des mélanges de ceux-ci.

7. Procédé selon une ou plusieurs des revendications 1 à 6, **caractérisé en ce que** le catalyseur au nickel contient une matière de support.

8. Procédé selon une ou plusieurs des revendications 1 à 7, **caractérisé en ce que** le catalyseur au nickel contient comme additifs des oxydes du sodium, du potassium, du magnésium, du calcium, du baryum, du zinc, de l'aluminium, du zirconium, du chrome ou des mélanges de ceux-ci.
